(19) Europäisches Patentamt
European Patent Office
Office européen des brevets

(11) Veröffentlichungsnummer : **0 083 556**
**B1**

(12) # EUROPÄISCHE PATENTSCHRIFT

(45) Veröffentlichungstag der Patentschrift : **20.08.86**

(51) Int. Cl.⁴ : **C 07 D213/64**, A 01 N 43/40

(21) Anmeldenummer : **82810568.4**

(22) Anmeldetag : **24.12.82**

(54) **Alpha-(4-(3'-Fluor-5'-halogen-pyridyl-2'-oxy)-phenoxy)-propionsäure derivate mit herbizider Wirkung.**

(30) Priorität : **31.12.81 CH 8372/81**

(43) Veröffentlichungstag der Anmeldung : **13.07.83 Patentblatt 83/28**

(45) Bekanntmachung des Hinweises auf die Patenterteilung : **20.08.86 Patentblatt 86/34**

(84) Benannte Vertragsstaaten : **BE CH DE FR GB IT LI NL**

(56) Entgegenhaltungen :
**EP-A- 0 001 473**
**EP-A- 0 003 313**
**EP-A- 0 008 624**
**EP-A- 0 021 453**
**EP-A- 0 050 097**
**FR-A- 2 419 285**

(73) Patentinhaber : **CIBA-GEIGY AG**
**Klybeckstrasse 141**
**CH-4002 Basel (CH)**

(72) Erfinder : **Schurter, Rolf, Dr.**
**Holzmattstrasse 45**
**CH-4102 Binningen (CH)**
Erfinder : **Rempfler, Hermann, Dr.**
**Brücklismattstrasse 16**
**CH-4107 Ettingen (CH)**

**EP 0 083 556 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue α-[4-(3'-Fluor-5'-halogenpyridyl-2'-oxy)-phenoxy]-propionsäurederivate mit herbizider Wirkung, deren Herstellung, Mittel welche diese Derivate als Wirkstoffe enthalten sowie deren Verwendung als Herbizide allgemien und speziell zur Bekämpfung von Unkräutern in Nutzpflanzenkulturen wie Getreiden, Reis, Mais, Soja, Zuckerrüben.

Die α-[4-(3'-Fluor-5'-halogen-pyridyl-2'-oxy)-phenoxy]-propionsäurederivate entsprechen der Formel I

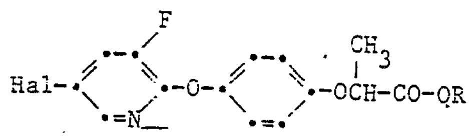

(I)

worin

Hal Halogen,

Q Sauerstoff oder Schwefel,

R Wasserstoff, ein Alkalimetallion, oder einen quaternären $C_1$-$C_4$ Alkylammoniumrest,

einen $C_1$-$C_6$ Alkylrest, der geradkettig oder verzweigt, unsubstituiert oder substituiert durch Halogen, Cyan, $C_1$-$C_4$ Alkoxy, $C_1$-$C_4$-Alkylcarbonyl, $C_1$-$C_4$ Alkoxycarbonyl, Carbamoyl oder Di-$C_1$-$C_4$ Alkylcarbamoyl ist,

einen $C_3$-$C_6$ Cycloalkylrest

einen $C_3$-$C_6$ Alkenylrest, der geradkettig oder verzweigt, unsubstituiert oder durch Halogen substituiert ist

einen $C_3$-$C_6$ Alkinylrest, der geradkettig oder verzweigt, unsubstituiert oder durch Halogen substituiert ist,

einen Rest

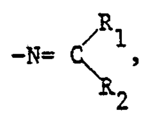

worin $R_1$ und $R_2$ einzeln je einen $C_1$-$C_4$ Alkylrest oder zusammen eine 4-5-gliedrige Methylenkette bilden, die durch $C_1$-$C_4$ Alkyl substituiert sein kann.

Die wichtigsten Vertreter der erfindungsgemässen α-[4-(3'-Fluor-5'-halogenpyridyl-2'-oxy)-phenoxy]-propionsäurederivate der Formel I sind dadurch gekennzeichnet, dass

Hal Chlor,

Q Sauerstoff

R Wasserstoff oder das Ion eines Alkalimetalles, -$C_1$-$C_6$-Alkyl, geradkettig oder verzweigt, oder einen Rest

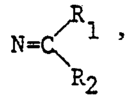

worin $R_1$ und $R_2$ einzeln je $C_1$-$C_4$ Alkyl bedeuten.

Aufgefallen sind die Verbindungen

α-[4-(5'-Chlor-3'-fluorpyridyl-2'-oxy)phenoxy]-propionsäure-methylester
α-[4-(5'-Chlor-3'-fluorpyridyl-2'-oxy)phenoxy]-propionsäure-n-propylester
α-[4-(5'-Chlor-3'-fluorpyridyl-2'-oxy)phenoxy]-propionsäure
α-[4-(5'-Chlor-3'-fluorpyridyl-2'-oxy)phenoxy]-propionsäure acetoxim ester

Die erfindungsgemässen α-[4-(3'-Fluor-5'-halogenpyridyl-2'-oxy)-phenoxy]-propionsäurederivate zeichnen sich durch eine gute Wirkung gegen mono- und einige dikotyle Unkräuter aus, vor allem wirken sie im Nachauflaufverfahren gegen widrige in Kulturen wie Getreide, Mais, Reis, Soja und Zuckerrüben vorkommende Unkräuter und Ungräser. Besonders wertvoll ist die Möglichkeit mit ihnen Ungräser zu bekämpfen, denen sonst nur schwer beizukommen ist, wie z. B. Avena fatua, Avena sterilis, Alopecurus myosuroides, Lolium perenne, Phalaris sp.

Bromus tectorum, verschiedene Setaria- und Panicum Arten. Die Wirkung tritt unter Feldbedingungen bereits bei niederen Aufwandmengen von weniger als 1 kg pro Hektar auf bei denen die Kulturen nicht oder nur ganz unbedeutend geschädigt werden.

Halogenpyridyloxy-α-phenoxy-propionsäurederivate sind in zahlreichen Publikationen beschrieben worden vgl. beispielsweise die DE-A 2 546 251, 2 649 706, 2 714 662, 2 715 284 sowie die EP-A 483 und 1473. Darin werden die erfindungsgemässen α-[4-(3'-Fluor-5'-halogenpyridyl-2'-oxy)-phenoxy]-proprionsäurederivate zum Teil auch in Erwägung gezogen und vom Umfang miterfasst. Ferner sind aus der EP-A 3 313 α-[4-(Pyridyl-2-oxy)phenoxy]-proprionsäure-aminoalkylester, aus der EP-A 21 453 α-[4-(5-Trifluormethylpyridyl-2-oxy)phenoxy]propionsäurederivate und aus der FR-A 2 419 285 4-[4-(5-Di- und Trifluormethylpyridyl-2-oxy)phenoxy]-valeriansäurederivate mit herbizider Wirkung bekannt geworden.

Die erfindungsgemässen Verbindungen sind jedoch bisher nicht hergestellt und geprüft worden. Sie eignen sich zur Bekämpfung von vor allem grasartigen Unkräutern (Monokotyledonen). Die Verbindungen der Formel I besitzen eine selektive herbizide Wirkung und können daher als selektive Unkrautmittel verwendet werden. Insbesondere eignen sich die Verbindungen der Formel I und die sie enthaltenden Mittel zur Bekämpfung von grasartigen Unkräuten.

Die erfindungsgemässen Verbindungen unterscheiden sich von den bekannten Halogenpyridyloxy-α-phenoxy-propionsäuren durch stärkere Wirkung und damit der Möglichkeit sie in geringeren Aufwandmengen einzusetzen.

Bei genügend grosser Aufwandmenge ist auch totalherbizide Wirkung vorhanden. Die Anwendung kann sowohl im Vorauflauf- wie im Nachauflaufverfahren vorgenommen werden. Dabei können die Aufwandmengen in weiten Grenzen schwanken, z. B. zwischen 0,05 bis 5 kg Wirkstoff pro Hektare.

Ferner besitzen die Verbindungen der Formel I günstige wachstumsregulierende Effekte. Sie eignen sich zur Hemmung der Pflanzenwachstums und hemmen insbesondere das Wachstum von Gräsern.

Als sehr aktiv haben sich diejenigen α-[4-(3'-Fluor-5'-halogenpyridyl-2'-oxy)-phenoxy]-propionsäurederivate der Formel I erwiesen, in denen entweder

Hal, Chlor, Brom oder Jod,

Q Sauerstoff oder Schwefel,

R Wasserstoff oder das Ion eines Alkalimetalles, -$C_1$-$C_6$ Alkyl, geradkettig oder verzweigt substituiert durch Halogen, Hydroxy, Cyan, Nitro, $C_1$-$C_4$ Alkoxy ($C_1$-$C_4$ alkoxy)$_n$-, $C_1$-$C_4$ Alkylamino, Di($C_1$-$C_4$ alkylamino), $C_1$-$C_4$ Alkoxycarbonyl, Carbamoyl oder Di($C_1$-$C_4$ Alkyl)carbamoyl und

n Null oder eine Zahl von 1 bis 5 bedeuten,

oder

X und Q die obige Bedeutung haben und

R einen $C_2$-$C_6$ Alkenyl oder Alkinylrest bedeutet der unsubstituiert oder durch Halogen, Hydroxyl oder $C_1$-$C_4$ Alkoxy substuiert ist, ferner diejenigen in denen

X und Q die obige Bedeutung haben und

R einen Iminoätherrest —$N=C(R_3)_2$ bedeutet, worin $R_3$ je einzeln $C_1$-$C_4$ Alkyl oder zusammen eine 4-5-gliedrige Polymethylenkette bedeuten,

speziell die α-[4-(3'-Fluor-5'-chlorpyridyl-2'-oxy)-phenoxy]-propionsäure, sowie deren Methyl- und Butylester.

Die Herstellung der neuen Verbindungen der Formel I erfolgt nach an sich bekannten Methoden.

Nach einem ersten dieser Verfahren setzt man ein 2,5-Dihalogen-3-fluorpyridin der Formel II

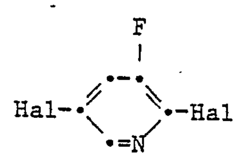

(II)

worin Hal Halogen bedeutet, in einem inerten Lösungs- oder Verdünnungsmittel, in Gegenwart der äquimolaren Menge einer Base mit einem 4-Hydroxyphenoxy-α-propionsäureester der Formel III um,

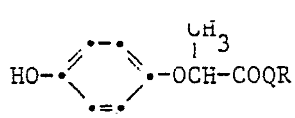

(III)

worin Q und R die unter Formel I gegebene Bedeutung haben.

Ein anderes Verfahren besteht darin, dass man ein 4-(3'-Fluor-5'-halogenpyridyl-2'-oxy)-phenol der Formel IV

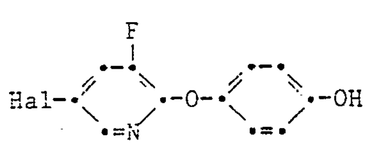

(IV)

worin Hal Halogen bedeutet, in einem inerten Lösungs- oder Verdünnungsmittel in Gegenwart der äquimolaren Menge einer Base mit einem α-Halogenpropionsäureester der Formel V umsetzt

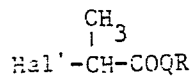 (V)

worin Hal' Chlor oder Brom bedeutet und Q und R die unter Formel I gegebene Bedeutung haben.

Ein weiteres Verfahren besteht darin, dass man ein α-[4-(3'-Fluor-5'-halogenpyridyl-2'-oxy)-phenoxy]-propionsäurehalogenid der Formel VI

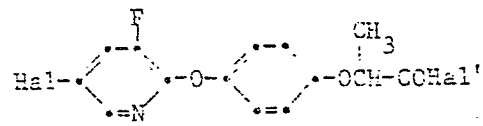 (VI)

worin Hal' Chlor oder Brom und Hal Halogen bedeutet, in einem inerten Lösungs- oder Verdünnungsmittel in Gegenwart der äquimolaren Menge einer Base mit einem Alkohol oder Thiol der Formel VII umsetzt,

HQR (VII),

worin Q und R die unter Formel I gegebene Bedeutung haben.

Zudem können die Verbindungen der Formel I auch hergestellt werden, indem man eine α-[4-(3'-Fluor-5'-halogenpyridyl-2'-oxy)-phenoxy]-propionsäure oder -thiopropionsäure der Formel IX,

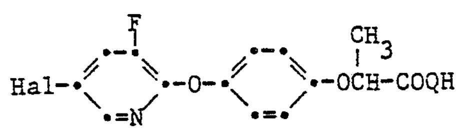 (IX)

worin Hal und Q die unter Formel I gegebene Bedeutung haben, in einem inerten Lösungs- oder Verdünnungsmittel, in Anwesenheit der äquimolaren Menge einer Base mit einem Halogenid der Formel X

Hal—R (X),

worin Hal Halogen bedeutet und R die unter Formel I gegebene Bedeutung hat, umsetzt.

Schliesslich besteht ein weiteres Verfahren darin, dass man einen α-[4-(3'-Amino-5'-halogenpyridyl-2'-oxy)-phenoxy]-propionsäureester der Formel XI

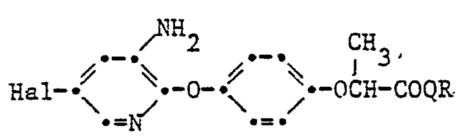 (XI)

worin Hal, Q und R die unter Formel I gegebene Bedeutung haben, nach bekannten Methoden in ein Diazoniumsalz und dieses weiter in die Fluorverbindungen überführt.

Ein Teil dieser Umsetzungen werden vorteilhafterweise in einem den Reaktionspartnern gegenüber inerten, organischen Lösungs- oder Verdünnungsmittel durchgeführt, wie z. B. einem Alkohol, Ester, Aether, Keton, Dimethylformamid, Dimethylsulfoxyd, Acetonitril, einem Aromaten wie Benzyl, Toluol etc..

Die Reaktionstemperaturen liegen zwischen —10 °C et + 150 °C, praktisch aber zwischen der Raumtemperatur und dem Siedepunkt des Lösungsmittels. Die Reaktionsdauer beträgt je nach dem gewählten Ausgangsstoff, dem Lösungsmittel und der Temperatur 1 Stunde bis ca. einen Tag.

Wo ein Halogenatom bei der Reaktion abgespalten wird, sollte die äquimolare Menge eines säurebindenden Mittels eingesetzt werden. Als solches eignet sich im Prinzip jede anorganische oder organische Base, wie z. B. NaOH, KOH, $NaHCO_3$, $K_2CO_3$, K-tert. Butylat, und Amine, wie Trimethylamin, Triäthylamin, Pyridin, 4-Dimethylaminopyridin, etc..

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxid etc. löslich sind. Sie sind nicht explosiv oder ätzend und ihre Handhabung bedarf keiner besonderer Vorsichtsmassnahmen.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen,

Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen fester oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl äther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxidertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemisch zu verstehen.

Geeignete anionische Tenside können sowohl sog. wasserlösliche Seifen wie wasserlösliche synthetische oberflächenaktive Verbindungen sein.

Als Seifen sind z. B. die Alkali-, Erdalkali- oder gegebenenfalls substituierten Ammoniumsalze von höheren Fettsäuren ($C_{10}$-$C_{20}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyl-taurinsalze zu erwähnen.

Haüfiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxy-alkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben :

« Mc Cutcheon's Detergents and Emulsifiers Annual »
MC Publishing Corp., Ringwood, New Jersey, 1979.
Sisely and Wood, « Encyclopedia of Surface Active Agents »,
Chemical Publishing Co., Inc. New York, 1964.

Diese Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die folgenden Beispiele beschreiben im Detail die Herstellung eines erfindungsgemässen α-[4-(3'-Fluor-5'-halogenpyridyl-2'-oxy)-phenoxy]-propionsäureesters der Formel I und solche Ester als Wirkstoffe enthaltende Mittel. Weitere in analoger Weise erhaltene erfindungsgemässe Ester sind in der sich dem Beispiel 1 anschliessenden Tabelle aufgeführt. Die Temperaturen sind in Grad Celsius angegeben, während sich Prozentangaben auf das Gewicht beziehen.

## Beispiel 1

Herstellung von α-[4-(3'-Fluor-5'-chlor-pyridyl-2'-oxy)-phenoxy]-propionsäuremethylester

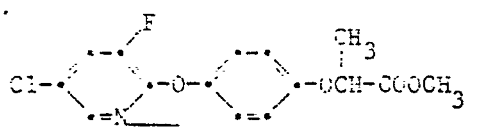

Verbindung No. 1

8,2 g (0.025 Mol) α-[4-(3'-Amino-5'-chlor-pyridyl-2'-oxy)-phenoxy]-propionsäuremethylester werden in 120 ml Borfluorwasserstoffsäure ($HBF_4$, 50 %) gelöst und auf 0 °C gekühlt. Dann werden 1.87 g (0.027 Mol) Natriumnitrit in 20 ml Wasser während einer Stunde zugetropft. Nach einer weiteren Stunde wird das Produkt abfiltriert, gewaschen und (bei Raumtemperatur und über Phosphorpentoxid) gut getrocknet. Das so erhaltene Diazonium-tetrafluoroborat wird während 10 Minuten auf 150 °C erwärmt. Der noch heisse Rückstand wird in Methanol gelöst und nach Zugabe von etwas Aktivkohle filtriert und am Rotationsverdampfer eingeengt. Nach Reinigung durch eine Kieselgelsäule (eluiert mit Methylenchlorid/Hexan 6 : 1) werden 5,5 g (67 % der Theorie) der Titelverbindung erhalten. Schmelzpunkt 63-64 °C.

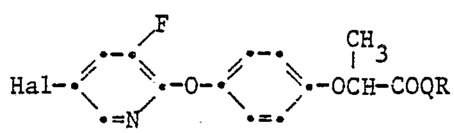

| Nr. | Hal | Q | R | physikal. Daten |
|---|---|---|---|---|
| 1 | Cl | O | $CH_3$ | Smp. 63-64°C |
| 2 | Br | O | $CH_3$ | |
| 3 | J | O | $CH_3$ | |
| 4 | Cl | O | $C_2H_5$ | |
| 5 | Br | S | $C_2H_5$ | |
| 6 | J | O | $C_2H_5$ | |
| 7 | Cl | O | $(CH_2)_2CH_3$ | Oel |
| 8 | Br | O | $(CH_2)_2CH_3$ | |
| 9 | J | O | $(CH_2)_2CH_3$ | |
| 10 | Cl | O | $CH(CH_3)_2$ | |

(Fortsetzung)

| Nr. | Hal | Q | R | physikal. Daten |
|---|---|---|---|---|
| 11 | Cl | S | $CH(CH_3)_2$ | |
| 12 | Br | O | $CH(CH_3)_2$ | |
| 13 | J | O | $CH(CH_3)_2$ | |
| 14 | Cl | O | $(CH_2)_3CH_3$ | |
| 15 | Br | O | $(CH_2)_3CH_3$ | |
| 16 | J | O | $(CH_2)_3CH_3$ | |
| 17 | Cl | O | $C_2H_4N(CH_3)_2$ | |
| 18 | Cl | O | $C_2H_4N(C_2H_5)_2$ | |
| 19 | Cl | S | $C_2H_4N(C_2H_5)_2$ | |
| 20 | Cl | O | $C_3H_6N(CH_3)_2$ | |
| 21 | Br | O | $C_2H_4N(C_2H_5)_2$ | |
| 22 | J | O | $C_2H_4N(C_2H_5)_2$ | |
| 23 | Cl | O | $C_2H_4N(C_2H_4OH)_2$ | |
| 24 | Br | O | $C_2H_4N(C_2H_4OH)_2$ | |
| 25 | Cl | O | $CH_2C{\equiv}CH$ | |
| 26 | Br | O | $CH_2C{\equiv}CH$ | |
| 27 | J | O | $CH_2C{\equiv}CH$ | |
| 28 | Cl | S | $CH_2C{\equiv}CH$ | |
| 29 | Br | S | $CH_2C{\equiv}CH$ | |
| 30 | J | S | $CH_2C{\equiv}CH$ | |
| 31 | Cl | O | $CH_2CH{=}CH_2$ | |
| 32 | Br | O | $CH_2CH{=}CH_2$ | |
| 33 | J | O | $CH_2CH{=}CH_2$ | |
| 34 | Cl | S | $CH_2CH{=}CH_2$ | |
| 35 | Br | S | $CH_2CH{=}CH_2$ | |
| 36 | J | S | $CH_2CH{=}CH_2$ | |
| 37 | Cl | O | $CH_2C(CH_3){=}CH_2$ | |
| 38 | Br | O | $CH_2C(CH_3){=}CH_2$ | |
| 39 | J | O | $CH_2C(CH_3){=}CH_2$ | |
| 40 | Cl | S | $CH_2C(CH_3){=}CH_2$ | |
| 41 | Br | S | $CH_2C(CH_3){=}CH_2$ | |
| 42 | J | S | $CH_2C(CH_3){=}CH_2$ | |
| 43 | Cl | O | $CH_2CN$ | |
| 44 | Cl | S | $CH_2CN$ | |
| 45 | Cl | O | $CH(CH_3)CN$ | |
| 46 | Cl | O | $N{=}C(CH_3)_2$ | Oel |

(Fortsetzung)

| Nr. | Hal | Q | R | physikal. Daten |
|---|---|---|---|---|
| 47 | Br | O | $N=C(CH_3)_2$ | |
| 48 | J | O | $N=C(CH_3)_2$ | |
| 49 | Cl | O | $N=C(C_2H_5)_2$ | |
| 50 | Br | O | $N=C(C_2H_5)_2$ | |
| 51 | J | O | $N=C(C_2H_5)_2$ | |
| 52 | Cl | O | $N=C(CH_3)C_2H_5$ | |
| 53 | Br | O | $N=C(CH_3)C_2H_5$ | |
| 54 | J | O | $N=C(CH_3)C_2H_5$ | |
| 55 | Cl | O | $CH_2-C\equiv C-CH_2Cl$ | |
| 56 | Cl | O | $CH_2-C(Br)=CH_2$ | |
| 57 | Cl | O | $CH_2-CH=CH-Cl$ | |
| 58 | Cl | O | $C(CH_3)_2CN$ | |
| 59 | Br | S | $CH_2-CH=CH-CH_3$ | |
| 60 | Br | S | $CH_2CH_2-CH=CH_2$ | |
| 61 | Cl | O | -N= 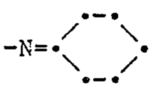 | |
| 62 | Br | O | -N= 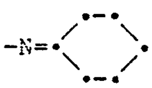 | |
| 63 | J | O | -N= 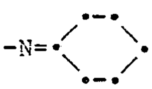 | |
| 64 | Cl | O | $CH_2COOCH_3$ | |
| 65 | Cl | S | $CH_2COOCH_3$ | |
| 66 | Br | O | $CH_2COOCH_3$ | |
| 67 | Br | S | $CH_2COOCH_3$ | |
| 68 | J | O | $CH_2COOCH_3$ | |
| 69 | J | S | $CH_2COOCH_3$ | |
| 70 | Cl | O | $CH(CH_3)COOC_2H_5$ | |
| 71 | Cl | S | $CH(CH_3)COOC_2H_5$ | |
| 72 | Br | O | $CH(CH_3)COOC_2H_5$ | |
| 73 | Br | S | $CH(CH_3)COOC_2H_5$ | |
| 74 | J | O | $CH(CH_3)COOC_2H_5$ | |
| 75 | J | S | $CH(CH_3)COOC_2H_5$ | |
| 76 | Cl | O | $CH_2CONH_2$ | |
| 77 | Cl | O | $CH_2CH_2COOCH_3$ | |
| 78 | Cl | O | $CH_2COOC_4H_9n$ | |
| 79 | J | O | $CH_2COOC_4H_9n$ | |

(Fortsetzung)

| Nr. | Hal | Q | R | physikal. Daten |
|---|---|---|---|---|
| 80 | Cl | S | $CH_2COOC_4H_9n$ | |
| 81 | Br | S | $CH_2COOCH(CH_3)_2$ | |
| 82 | Cl | S | $CH_2CON(CH_3)_2$ | |
| 83 | Cl | O | H | Smp. 95 - 97°C |
| 84 | Br | O | H | |
| 85 | J | O | H | |
| 86 | Cl | O | Na | |
| 87 | Br | O | Na | |
| 88 | J | O | Na | |

Beispiel 2

Herstellung einer Formulierung mit flüssigen Wirkstoffen der Formel I (% = Gewichtsprozent)

| Emulsions-Konzentrate | a) | b) | c) |
|---|---|---|---|
| α-[4-(3'-Fluor-5'-halogenpyridyl-2'-oxy)-phenoxy]-propionsäuremethylester | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenoyl-polyäthylenglykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| Lösungen | a) | b) | c) | d) |
|---|---|---|---|---|
| Wirkstoff gemäss Formel I | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenze 160-190°C) | - | - | 94% | - |

Die Lösungen sind zur Anwendung in Form kleinster Tropfen geeignet.

| Granulate | a) | b) |
|---|---|---|
| Wirkstoff gemäss Formel I | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| Stäubemittel | a) | b) |
|---|---|---|
| Wirkstoff gemäss Formel I | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| Spritzpulver | a) | b) |
|---|---|---|
| Wirkstoff der Formel I | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Emulsions-Konzentrat

| | |
|---|---|
| Wirkstoff der Formel I | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff der Formel I | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff der Formel I | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Umhüllungs-Granulat

| | |
|---|---|
| Wirkstoff der Formel I | 3 % |
| Polyäthylenglykol (M G 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Suspensions-Konzentrat

| | |
|---|---|
| Wirkstoff der Formel I | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %-ige wässrige Formaldehyd-Lösung | 0,2 % |
| Silikonöl in Form einer 75 %-igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 4 : Prüfung der herbiziden Wirkung.

Pre-emergente Herbizid-Wirkung (Keimhemmung)

Im Gewächshaus wird unmittelbar nach der Einsaat der Versuchspflanzen in Töpfe die Erdoberfläche mit einer wässrigen Dispersion des Wirkstoffes erhalten aus einem 25 %-igen Emulsionskonzentrat oder aus einem 25 %-igen Spritzpulver mit Wirkstoffen, die wegen ungenügender Löslichkeit nicht als Emulsionskonzentrat hergestellt werden können, gespritzt. Es werden verschiedene Konzentrationen angewendet und die Menge Wirkstoff wird in kg pro Hektar ausgerechnet. Die Töpfe werden dann im Gewächshaus bei 22-25 °C und 50-70 % relativer Luftfeuchtigkeit gehalten und regelmässig bewässert. Der Versuch wird nach 3 Wochen ausgewertet.

Der Zustand der Pflanzen wird gemäss folgendem Schema bewertet :

9 Pflanze gedeiht wie unbehandelte Kontrolle

6-8 leichte Schäden, die Pflanze kann sich erholen

4-5 mittlere Schäden, Kummerwuchs

2-3 schwere Schäden

1 Pflanze abgestorben oder hat nicht gekeimt

Die Resultate sind untenstehend zusammengefasst :

| Verbindung | 1 | 7 | 46 | 83 | A | B |
|---|---|---|---|---|---|---|
| Aufwandmenge kg/ha | 1 $\frac{1}{2}$ $\frac{1}{4}$ | 1 $\frac{1}{2}$ $\frac{1}{4}$ | 1 $\frac{1}{2}$ $\frac{1}{4}$ | 1 $\frac{1}{2}$ $\frac{1}{4}$ | 1 $\frac{1}{2}$ $\frac{1}{4}$ | 1 $\frac{1}{2}$ $\frac{1}{4}$ |
| Pflanze | | | | | | |
| Weizen | 2 6 7 | 4 6 8 | 4 6 8 | 4 6 8 | 8 9 9 | 9 9 9 |
| Soja | 9 9 9 | 8 9 9 | 9 9 9 | 8 8 8 | 7 9 9 | 9 9 9 |
| Baumwolle | 9 9 9 | 9 9 9 | 9 9 9 | 8 9 9 | 9 9 9 | 9 9 9 |
| Zuckerrübe | 9 9 9 | 7 8 8 | 9 9 9 | 8 9 9 | 9 9 9 | 9 9 9 |
| Avena fatua | 1 2 2 | 2 2 3 | 2 3 3 | 3 3 3 | 6 9 9 | 1 2 2 |
| Bromus tectorum | 1 1 1 | 2 3 3 | 2 3 3 | 3 3 3 | 4 8 9 | 6 8 9 |
| Alopecurus myos. | 1 1 1 | 1 1 2 | 1 2 2 | 2 2 2 | 3 9 9 | 1 2 2 |
| Digitaria sang. | 1 1 1 | 1 1 2 | 1 1 2 | 1 1 2 | 2 2 6 | 1 1 2 |

(Fortsetzung)

| Verbindung | 1 | 7 | 46 | 83 | A | B |
|---|---|---|---|---|---|---|
| Aufwandmenge kg/ha | $1\ \frac{1}{2}\ \frac{1}{4}$ | $1\ \frac{1}{2}\ \frac{1}{4}$ | $1\ \frac{1}{2}\ \frac{1}{4}$ | $1\ \frac{1}{2}\ \frac{1}{4}$ | $1\ \frac{1}{2}\ \frac{1}{4}$ | $1\ \frac{1}{2}\ \frac{1}{4}$ |
| Echinochloa c.g. | 1 1 1 | 1 1 2 | 1 1 3 | 1 1 1 | 2 6 9 | 1 1 1 |
| Sorghum halepense | 1 1 1 | 1 2 3 | 2 3 3 | 1 2 2 | 4 7 9 | 1 1 2 |
| Rottboellia exaltata | 1 1 2 | 1 1 2 | 2 3 3 | 2 2 3 | 2 4 9 | 1 1 1 |

Die Verbindung A ist α-[4-(3,5-Dichlorpyridyl-2-oxy)-phenoxy]-propionsäuremethylester, B ist α-[4-(3,5-Dichloropyridyl-2-oxy)-phenoxy]-priopionsäure, welche aus der DE-OS 2 546 251 (USP 4 046 553) bekannt sind.

Post-emergente Herbizid-Wirkung (Kontaktherbizid)

Eine grössere Anzahl Unkräuter und Kulturpflanzen, sowohl monokotyle wie dicotyle, wurden im Gewächshaus in Töpfen gezogen und nach dem Auflaufen (im 4- bis 6-Blattstadium) mit einer wässerigen Wirkstoffdispersion in verschiedenen Dosierungen, ausgedrückt in kg Wirksubstanz pro Hektare, auf die Pflanzen gespritzt und diese bei 24-26 °C und 45-60 % relativer Luftfeuchtigkeit gehalten. Der Versuch wird 2 Wochen nach der Behandlung ausgewertet. Die Resultate sind untenstehend zusammengefasst :

| Verbindung | 1 | 7 | 46 | 83 | A | B |
|---|---|---|---|---|---|---|
| Aufwandmenge kg/ha | $\frac{1}{2}\ \frac{1}{4}\ \frac{1}{8}$ | $\frac{1}{2}\ \frac{1}{4}\ \frac{1}{8}$ | | | $\frac{1}{2}\ \frac{1}{4}\ \frac{1}{8}$ | $\frac{1}{2}\ \frac{1}{4}\ \frac{1}{8}$ |
| Pflanze | | | | | | |
| Weizen | 2 3 8 | 3 8 9 | 4 6 9 | 3 5 5 | 9 9 9 | 9 9 9 |
| Soja | 8 8 9 | 9 9 9 | 9 9 9 | 8 9 9 | 9 9 9 | 9 9 9 |
| Baumwolle | 6 7 8 | 9 9 9 | 8 9 9 | 8 9 9 | 9 9 9 | 9 9 9 |
| Zuckerrübe | 7 8 8 | 9 9 9 | 8 9 9 | 8 8 9 | 9 9 9 | 9 [illegible] 9 |
| Avena fatua | 1 1 1 | 2 3 4 | 1 2 2 | 1 1 1 | 4 8 9 | 9 9 9 |
| Bromus tectorum | 4 6 7 | 2 2 2 | 1 1 1 | 2 4 6 | 9 9 9 | 6 9 9 |
| Alopecurus myos. | 1 2 2 | 2 3 4 | 2 2 2 | 1 1 2 | 3 5 8 | 6 9 9 |
| Digitaria sang. | 1 2 2 | 1 1 2 | 1 1 1 | 1 2 2 | 1 2 2 | 2 2 7 |
| Echinochloa c.g. | 1 1 1 | 1 1 4 | 1 1 1 | 1 1 2 | 1 1 2 | 3 7 7 |
| Sorghum halepense | 1 1 1 | 2 2 3 | 2 2 4 | 1 1 1 | 1 2 5 | 7 9 9 |

In diesen Versuchen zeigen die erfindungsgemässen Verbindungen eine bessere Kontrolle der Unkräuter als die bekannten.

Wuchshemmung bei Gräsern

In Kunststoffschalen mit Erde-Torf-Sand-Gemisch (6 : 3 : 1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussaat und 1 Tag nach dem letzten Schnitt mit einer wässerigen Spritzbrühe der Verbindungen der Formel I bespritzt. Die Wirkstoffmenge betrug umgerechnet 0,05-2 kg Aktivsubstanz pro Hektar. 10 und 21 Tage nach der Applikation wurde das Wachstum der Gräser mit derjenigen einer umbehandelten Kontrolle verglichen. Die Verbindungen 1, 7, 46 und 83 verminderten bei 0,05 kg pro Hektare das Wachstum der Gräser um 18-32 %.

**Patentansprüche**

1. α-[4-(3′-Fluor-5′-halogenpyridyl-2′-oxy)-phenoxy]-propionsäurederivate der Formel I

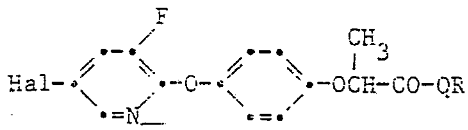

(I)

worin

Hal Halogen

Q Sauerstoff oder Schwefel,

R Wasserstoff, ein Alkalimetallion oder einen quaternären $C_1$-$C_4$ Alkylammoniumrest,

einen $C_1$-$C_6$-Alkylrest, der geradkettig oder verzweigt, unsubstituiert oder substituiert durch Halogen, Cyan, $C_1$-$C_4$-Alkoxy, $C_1$-$C_4$-Alkylcarbonyl, $C_1$-$C_4$ Alkoxycarbonyl, Carbamoyl, oder Di-$C_1$-$C_4$-Alkylcarbamoylrest, ist,

einen $C_3$-$C_6$-Cycloalkylrest,

einen $C_3$-$C_6$-Alkenylrest, der geradkettig oder verzweigt, unsubstituiert oder durch Halogen substituiert ist,

einen $C_3$-$C_6$-Alkinylrest, der geradkettig oder verzweigt, unsubstituiert oder durch Halogen substituiert ist,

einen Rest

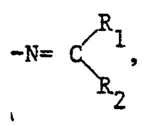

worin $R_1$ und $R_2$ einzeln je einen $C_1$-$C_4$-Alkylrest oder zusammen eine 4-5-gliedrige Methylenkette bilden, die durch $C_1$-$C_4$-Alkyl substituiert sein kann.

2. Die α-[4-(3′-Fluor-5′-halogenpyridyl-2′-oxy)-phenoxy]-propionsäurederivate gemäss Formel I, Anspruch 1, worin Hal Chlor, Q Sauerstoff und R Wasserstoff, das Ion eines Alkalimetalles ; $C_1$-$C_6$-Akyl, geradlinig oder verzweigt, oder einen Rest

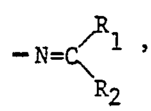

worin $R_1$ und $R_2$ einzeln je einen $C_1$-$C_4$-Alkylrest bedeuten.

3. α-[4-(3′-Fluor-5′-chlorpyridyl-2′-oxy)-phenoxy]-propionsäure gemäss Anspruch 1.

4. α-[4-(3′-Fluor-5′-chlorpyridyl-2′-oxy)-phenoxy]-propionsäure-methylester gemäss Anspruch 1.

5. α-[4-(3′-Fluor-5′-chlorpyridyl-2′-oxy)-phenoxy]-propionsäure-n-propylester gemäss Anspruch 1.

6. α-[4-(3′-Fluor-5′-chlorpyridyl-2′-oxy)-phenoxy]-propionsäureacetoximester gemäss Anspruch 1.

7. Verfahren zur Herstellung der α-[4-(3′-Fluor-5′-halogenpyridyl-2′-oxy)-phenoxy]-propionsäurederivate der Formel I, Anspruch 1, dadurch gekennzeichnet, dass man ein 2,5-Dihalogen-3-fluorpyridin der Formel II

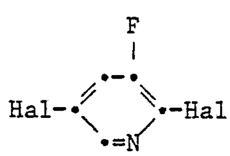

(II)

worin Hal Halogen bedeutet in einem inerten Lösungs- oder Verdünnungsmittel, in Gegenwart der äquimolaren Menge einer Base mit einem 4-Hydroxy-phenoxy-α-propionsäureester der Formel III umsetzt,

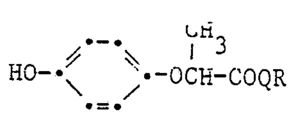

(III)

worin Q und R die unter Formel I gegebene Bedeutung haben.

8. Verfahren zur Herstellung der α-[4-(3′-Fluor-5′-halogenpyridyl-2′-oxy)-phenoxy]-propionäsureester der Formel I, Anspruch 1, dadurch gekennzeichnet, dass man ein 4-(3′-Fluor-5′-halogenpyridyl-2′-oxy)-phenol der Formel IV

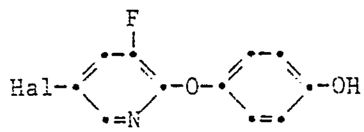

(IV)

worin Hal Halogen bedeutet, in einem inerten organischen Lösungsmittel, in Anwesenheit der äquimolaren Menge einer Base, mit einem α-Halogenpropionsäureester der Formel V umsetzt,

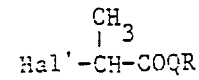

(V)

worin Hal' Chlor oder Brom bedeutet und Q und R die unter Formel I, Anspruch 1 gegebene Bedeutung haben.

9. Verfahren zur Herstellung der α-[4-(3'-Fluor-5'-halogenpyridyl-2'-oxy)-phenoxy]-propionsäureester der Formel I, Anspruch 1, dadurch gekennzeichnet, dass man ein α-[4-(3'-Fluor-5'-halogenpyridyl-2'-oxy)-phenoxy]-propionsäurehalid der Formel VI,

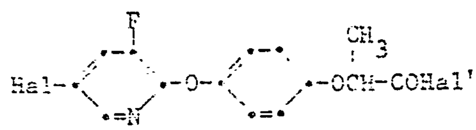

(VI)

worin Hal' Chlor oder Brom und Hal Halogen bedeutet, in einem inerten organischen Lösungs- oder Verdünnungsmittel in Gegenwart der äquimolaren Menge einer Base, mit einem Alkohol oder Thiol der Formel VII umsetzt

HQR (VII),

worin Q und R die unter Formel I, Anspruch 1 gegebene Bedeutung haben.

10. Verfahren zur Herstellung der α-[4-(3'-Fluor-5'-halogenpyridyl-2'-oxy)-phenoxy]-propionsäureester der Formel I, Anspruch 1, dadurch gekennzeichnet, dass eine α-[4-(3'-Fluor-5'-halogenpyridyl-2'-oxy)-phenoxy]-propionsäure oder -thiopropionsäure der Formel IX,

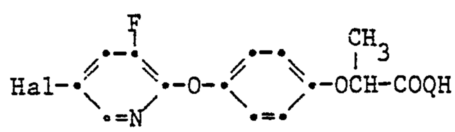

(IX)

worin Q Sauerstoff oder Schwefel und Hal Halogen bedeuten, in einem inerten organischen Lösungs- oder Verdünnungsmittel, in Gegenwart der äquimolaren Menge einer Base, mit einem Halogenid der Formel X umsetzt,

Hal—R (X)

worin Hal Halogen bedeutet und R die unter Formel I, Anspruch 1 gegebenen Bedeutung hat.

11. Verfahren zur Herstellung der α-[4-(3'-Fluor-5'-halogenpyridyl-2'-oxy)-phenoxy]-propionsäurederivate der Formel I, Anspruch 1, dadurch gekennzeichnet, dass ein α-[4-(3'-Amino-5'-halogenpyridyl-2'-oxy)-phenoxy]-propionsäureester der Formel XI

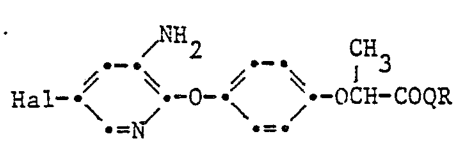

(XI)

worin Hal, Q und R die unter Formel I, Anspruch 1 gegebenen Bedeutungen haben, in ein Diazoniumsalz und dieses weiter in die Fluorverbindung überführt wird.

12. Herbizides und das Pflanzenwachstum hemmendes Mittel, dadurch gekennzeichnet, dass es als Wirkstoff mindestens einen α-[4-(3'-Fluor-5'-halogenpyridyl-2'-oxy)-phenoxy]-propionsäureester der Formel I, Anspruch 1, enthält.

13. Die Verwendung der α-[4-(3'-Fluor-5'-halogenpyridyl-2'-oxy)-phenoxy]-propionsäureester der Formel I, Anspruch 1 oder sie enthaltender Mittel zur Bekämpfung von grasartigen Unkräutern.

14. Die Verwendung der α-[4-(3'-Fluor-5'-halogenpyridyl-2'-oxy)-phenoxy]-propionsäureester der Formel I, Anspruch 1 oder sie enthaltender Mittel, als selektive Unkrautmittel in Kulturpflanzungen.

15. Die Verwendung der α-[4-(3'-Fluor-5'-halogenpyridyl-2'-oxy)-phenoxy]-propionsäureester der Formel I, Anspruch 1, oder sie enthaltender Mittel zur Hemmung des Pflanzen-, insbesondere des Gräserwuchses.

**Claims**

1. An α-[4-(3'-fluoro-5'-halopyridyl-2'-oxy)phenoxy]-propionic acid derivative of the formula I

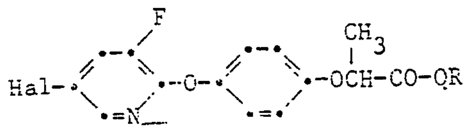

(I)

wherein
Hal is halogen
Q is oxygen or sulfur,
R is hydrogen, an alkali metal ion, or a quaternary $C_1$-$C_4$-alkylammonium group,
a $C_1$-$C_6$-alkyl group which is straight chain or branched and is unsubstituted or substituted by halogen, cyano, $C_1$-$C_4$-alkoxy, $C_1$-$C_4$-alkylcarbonyl, $C_1$-$C_4$-alkoxycarbonyl, carbamoyl or di-$C_1$-$C_4$-alkylcarbamoyl,
a $C_3$-$C_6$-cycloalkyl group,
a $C_3$-$C_6$-alkenyl group which is straight chain or branched and is unsubstituted of substituted by halogen,
a $C_3$-$C_6$-alkynyl group, which is straight chain or branched and is unsubstituted or substituted by halogen,
a group

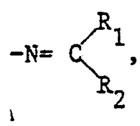

wherein $R_1$ and $R_2$ separately are each a $C_1$-$C_4$-alkyl group, or together form a 4- or 5-membered methylene chain which can be substituted by $C_1$-$C_4$-alkyl.

2. An α-[4-(3'-fluoro-5'-halopyridyl-2'-oxy)phenoxy]-propionic acid derivative according to formula I, claim 1 wherein Hal is chlorine, Q is oxygen, and R is hydrogen, the ion of an alkali metal, $C_1$-$C_6$-alkyl which is straight-chain or branched, or a group

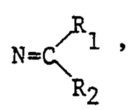

wherein each of $R_1$ and $R_2$ separately is $C_1$-$C_4$-alkyl.

3. α-[4-(3'-Fluoro-5'-chloropyridyl-2'-oxy)phenoxy]-propionic acid according to claim 1.

4. α-[4-(3'-Fluoro-5'-chloropyridyl-2'-oxy)phenoxy]-propionic acid methyl ester according to claim 1.

5. α-[4-(3'-Fluoro-5'-chloropyridyl-2'-oxy)phenoxy]-propionic acid n-propyl ester according to claim 1.

6. α-[4-(3'-Fluoro-5'-chloropyridyl-2'-oxy)phenoxy]-propionic acid acetoxime ester according to claim 1.

7. A process for the preparation of an α-[4-(3'-fluoro-5'-halopyridyl-2'-oxy)phenoxy]propionic acid derivative of the formula I, claim 1, which process comprises reacting a 2-halopyridine of the formula II

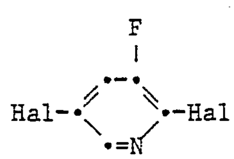

(II)

wherein Hal is halogen, in an inert solvent or diluent and in the presence of the equimolar amount of a base, with a 4-hydroxyphenoxy-α-propionic acid ester of the formula III

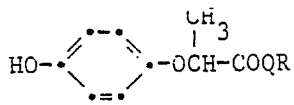 (III)

wherein Q and R have the meanings defined under the formula I.

8. A process for the preparation of an α-[4-(3'-fluoro-5'-halopyridyl-2'-oxy)phenoxy]propionic acid ester of the formula I, claim 1, which process comprises reacting a 4-(3'-fluoro-5'-halopyridyl-2'-oxy)phenol of the formula IV

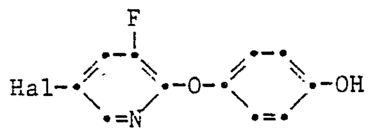 (IV)

wherein Hal is halogen, in an inert organic solvent or diluent and in the presence of the equimolar amount of a base, with an α-halopropionic acid ester of the formula V

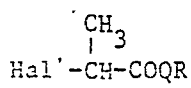 (V)

wherein Hal' is chlorine or bromine, and Q and R have the meanings defined under the formula I, claim 1.

9. A process for the preparation of an α-[4-(3'-fluoro-5'-halopyridyl-2'-oxy)phenoxy]propionic acid ester of the formula I, claim 1, which process comprises reacting an α-[4-(3'-fluoro-5'-halopyridyl-2'-oxy)phenoxy]propionic acid halide of the formula VI

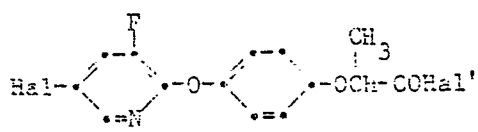 (VI)

wherein Hal' is chlorine or bromine and Hal is halogen, in an inert organic solvent or diluen,t and in the presence of the equimolar amount of a base, with an alcohol or thiol of the formula VII

HQR (VII)

wherein Q and R have the meanings defined under the formula I, claim 1.

10. A process for the preparation of an α-[4-(3'-fluoro-5'-halopyridyl-2'-oxy)phenoxy]propionic acid ester of the formula I, claim 1, which process comprises reacting an α-[4-(3'-fluoro-5'-halopyridyl-2'-oxy)phenoxy]propionic acid or -thiopropionic acid of the formula IX

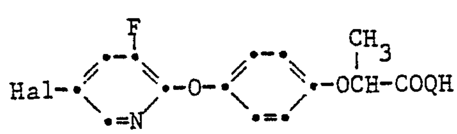 (IX)

wherein Q is oxygen or sulfur, and Hal is halogen, in an inert organic solvent or diluent and in the presence of the equimolar amount of a base, with a halide of the formula X

Hal—R (X)

wherein Hal is halogen, and R has the meaning defined und under the formula I, claim 1.

11. A process for the preparation of an α-[4-(3'-fluoro-5'-halopyridyl-2'-oxy)phenoxy]propionic acid derivative of the formula I, claim 1, which process comprises converting an α-[4-(3'-amino-5'-halopyridyl-2'-oxy)phenoxy]propionic acid ester of the formula XI

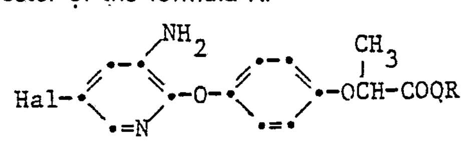 (XI)

wherein Hal, Q and R have the meanings defined under the formula I, claim 1, into.a diazonium salt, and converting said salt further into the fluorine compound.

12. A herbicidal and plant growth regulating composition containing, as active ingredient, at least one α-[4-(3'-fluoro-5'-halopyridyl-2'-oxy)phenoxy]propionic acid ester of the formula I, claim 1.

13. A method of selectively controlling gramineous weeds, which method comprises applying thereto or to the locus thereof a herbicidally effective amount of an α-[4-(3'-fluoro-5'-halopyridyl-2'-oxy)phenoxy]propionic acid ester of the formula I, claim 1, or of a composition containing this compound as active ingredient.

14. A method of selectively controlling weeds in cultivated crops, which method comprises applying thereto or to the locus thereof a herbicidally effective amount of an α-[4-(3'-fluoro-5'-halopyridyl-2'-oxy)phenoxy]propionic acid ester of the formula I, claim 1, or of a composition containing this compound as active ingredient.

15. A method of reducing the growth of plants, in particular the growth of grasses, which method comprises applying thereto or to the locus thereof an effective amount of an α-[4-(3'-fluoro-5'-halopyridyl-2'-oxy)phenoxy]propionic acid ester of the formula I, claim 1, or of a composition containing this compound as active ingredient.

**Revendications**

1. Dérivés d'acides α-[4-(3'-fluoro-5'-halogénopyridyl-2'-oxy)-phénoxy]-propioniques de formule I

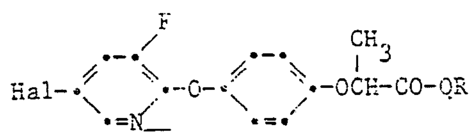

(I)

dans laquelle

Hal représente un halogène,

Q représente l'oxygène ou le soufre,

R représente l'hydrogène, un ion de métal alcalin ou groupe quaternaire alkylammonium en $C_1$-$C_4$,

un groupe alkyle en $C_1$-$C_6$ à chaîne droite ou ramifiée non substitué ou substitué par un ou des substituants choisis parmi halogène, cyano, alcoxy en $C_1$-$C_4$, (alkyle en $C_1$-$C_4$)-carbonyle, (alcoxy en $C_1$-$C_4$)-carbonyle, carbamoyle ou di-(alkyle en $C_1$-$C_4$)-carbamoyle,

un groupe cycloalkyle en $C_3$-$C_6$,

un groupe alcényle en $C_3$-$C_6$ à chaîne droite ou ramifiée, non substitué ou substitué par un ou des halogènes,

un groupe alcynyle en $C_3$-$C_6$ à chaîne droite ou ramifiée non substitué ou substitué par un ou des halogènes,

un groupe

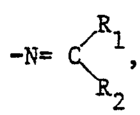

dans lequel $R_1$ et $R_2$ représentent chacun, individuellement, un groupe alkyle en $C_1$-$C_4$ ou bien forment ensemble une chaîne méthylénique à 4-5 chaînons qui peut être substituée par des groupes alkyle en $C_1$-$C_4$.

2. Les dérivés d'acides α-[4-(3'-fluoro-5'-halogénopyridyl-2'-oxy)-phénoxy]-propioniques de formule I, revendication 1, dans lesquels Hal représente le chlore, Q représente l'oxygène et R représente l'hydrogène, l'ion d'un métal alcalin ; un groupe alkyle en $C_1$-$C_6$ à chaîne droite ou ramifiée ou un groupe

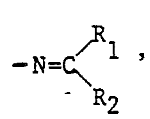

dans lequel $R_1$ et $R_2$ représentent chacun, individuellement, un groupe alkyle en $C_1$-$C_4$.

3. L'acide α-[4-(3'-fluoro-5'-chloropyridyl-2'-oxy)-phénoxy]-propionique selon la revendication 1.

4. L'α-[4-(3'-fluoro-5'-chloropyridyl-2'-oxy)-phénoxy]-propionate de méthyle selon la revendication 1.

5. L'α-[4-(3'-fluoro-5'-chloropyridyl-2'-oxy)-phénoxy]-propionate de n-propyle selon la revendication 1.

6. L'ester α-[4-(3'-fluoro-5'-chloropyridyl-2'-oxy)-phénoxy]-propionique de l'acétoxime selon la revendication 1.

7. Procédé de préparation des dérivés d'acides α-[4-(3'-fluoro-5'-halogénopyridyl-2'-oxy)-phénoxy]-propioniques de formule I, revendication 1, caractérisé en ce que l'on fait réagir une 2,5-dihalogéno-3-fluoropyridine de formule II

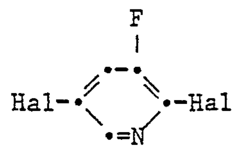

(II)

dans laquelle Hal représente un halogène, dans un solvant ou diluant inerte, en présence de la quantité équimoléculaire d'une base, avec un ester 4-hydroxy-phénoxy-α-propionique de formule III

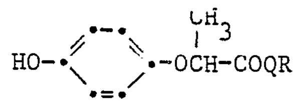

(III)

dans laquelle Q et R ont les significations indiquées en référence à la formule I.

8. Procédé de préparation des esters d'acides [4-(3'-fluoro-5'-halogénopyridyl-2'-oxy)-phénoxy]-propioniques de formule I, revendication 1, caractérisé en ce que l'on fait réagir un 4-(3'-fluoro-5'-halogénopyridyl-2'-oxy)-phénol de formule IV

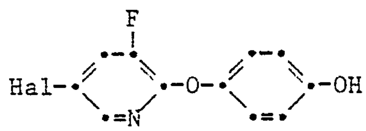

(IV)

dans laquelle Hal représente un halogène , dans un solvant organique inerte, en présence de la quantité équimoléculaire d'une base, avec un ester α-halogénopropionique de formule V

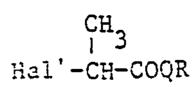

(V)

dans laquelle Hal' représente le chlore ou le brome et Q et R ont les significations indiquées en référence à la formule I, revendication 1.

9. Procédé de préparation des esters α-[4-(3'-fluoro-5'-halogénopyridyl-2'-oxy)-phénoxy]-propioniques de formule I, revendication 1, caractérisé en ce que l'on fait réagir un halogénure d'acide α-[4-(3'-fluoro-5'-halogénopyridyl-2'-oxy)-phénoxy]-propionique de formule VI

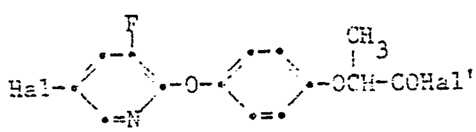

(VI)

dans laquelle Hal' représente le chlore ou le brome et Hal un halogène, dans un solvant ou diluant organique inerte, en présence de la quantité équimoléculaire d'une base, avec un alcool ou un thiol de formule VII

HQR (VII)

dans laquelle Q et R ont les significations indiquées en référence à la formule I, revendication 1.

10. Procédé de préparation des esters α-[4-(3'-fluoro-5'-halogénopyridyl-2'-oxy)-phénoxy]-propioniques de formule I, revendication 1, caractérisé en ce que l'on fait réagir un acide α-[4-(3'-fluoro-5'-halogénopyridyl-2'-oxy)-phénoxy]-propionique ou -thiopropionique de formule IX

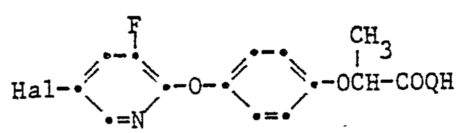

(IX)

dans laquelle Q représente l'oxygène ou le soufre et Hal un halogène, dans un solvant ou diluant organique inerte, en présence de la quantité équimoléculaire d'une base, avec un halogénure de formule X

Hal—R (X)

dans laquelle Hal représente un halogène et R a les significations indiquées en référence à la formule I, revendication 1.

11. Procédé de préparation des dérivés d'acides α-[4-(3'-fluoro-5'-halogénopyridyl-2'-oxy)-phénoxy]-propioniques de formule I, revendication 1, caractérisé en ce que l'on convertit un ester α-[4-(3'-amino-5'-halogénopyridyl-2'-oxy)-phénoxy]-propionique de formule XI

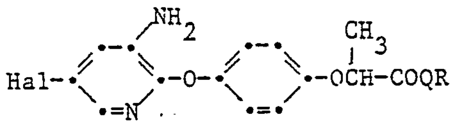

(XI)

dans laquelle Hal, Q et R ont les significations indiquées en référence à la formule I, revendication 1, en un sel de diazonium qu'on convertit ensuite en le composé fluoré.

12. Produit herbicide et inhibiteur de la croissance des végétaux, caractérisé en ce qu'il contient en tant que substance active au moins un ester α-[4-(3'-fluoro-5'-halogénopyridyl-2'-oxy)-phénoxy]-propionique de formule I, revendication 1.

13. Utilisation des esters α-[4-(3'-fluoro-5'-halogénopyridyl-2'-oxy)-phénoxy]-propioniques de formule I, revendication 1, ou de produits en contenant, dans la lutte contre les mauvaises herbes du genre graminées.

14. Utilisation des esters α-[4-(3'-fluoro-5'-halogénopyridyl-2'-oxy)-phénoxy]-propioniques de formule I, revendication 1, ou de produits en contenant, en tant qu'herbicides sélectifs dans des cultures.

15. Utilisation des esters α-[4-(3'-fluoro-5'-halogénopyridyl-2'-oxy)-phénoxy]-propioniques de formule I, revendication 1, ou de produits en contenant, pour l'inhibition de la croissance des végétaux, en particulier de la croissance des graminées.